# EUROPEAN PATENT APPLICATION

(11) **EP 4 721 729 A1**
(43) Date of publication of application: **08.04.2026**
(21) Application number: 24815946.9
(22) Date of filing: 03.06.2024
(51) Int. Cl.: A61K 9/16, A61K 38/22, A61P 3/10, A61P 3/04, A61P 1/16

(54) **SUSTAINED-RELEASE MICROSPHERE PREPARATION COMPRISING TIRZEPATIDE OR PHARMACEUTICALLY ACCEPTABLE SALT THEREOF AND METHOD FOR PREPARING SAME**

(30) Priority: 02.06.2023 KR 20230071359
(71) Applicant: G2GBIO, Inc., Cheongju-si, Chungcheongbuk-do 28161 (KR)
(72) Inventor: LEE, Heeyong, Daejeon 34032 (KR); LEE, Jinwoo, Sejong-si 30144 (KR); KIM, Hyemin, Cheongju-si Chungcheongbuk-do 28222 (KR); KIM, Boyeon, Cheongju-si Chungcheongbuk-do 28222 (KR); SEOL, Eunyoung, Daejeon 34080 (KR)
(74) Representative: Ricker, Mathias
(86) International application number: PCT/KR2024/007594
(87) International publication number: WO 2024/248572

(57) **Abstract**

The present invention relates to a pharmaceutical composition useful for prevention or treatment of diabetes, beta-cell dysfunction, hypertension, hyperlipidemia, obesity, and non-alcoholic steatohepatitis because it does not have a rapid initial burst of a drug, includes a drug with high content relative to a particle size, and minimizes patient pain and an inflammatory response that may occur when administered to a human body due to high bioavailability by including a sustained-release microsphere formed of tirzepatide or a pharmaceutically acceptable salt thereof, an initial burst inhibiting agent, and a biodegradable polymer.

## Description

### [Technical Field]

The present invention relates to a pharmaceutical composition including a sustained-release microsphere that includes a high content of tirzepatide or a pharmaceutically acceptable salt thereof and a method of preparing the sustained-release microsphere.

### [Background Art]

Tirzepatide sold under a brand name Mounjaro is an antidiabetic drug used to treat type 2 diabetes. The tirzepatide is administered once a week via subcutaneous injection.

Most common side effects of the tirzepatide include nausea, vomiting, diarrhea, decreased appetite, constipation, upper abdominal discomfort, abdominal pain, etc.

Glucagon-like peptide-1 (GLP-1) and glucose-dependent insulinotropic polypeptide (GIP) are hormones involved in blood glucose regulation. After eating food, these hormones are secreted from cells of an intestine and triggers a secretion of the insulin. The tirzepatide is known as a GIP analogue that improves the blood glucose regulation by activating both GLP-1 and GIP receptors.

The tirzepatide was approved for medical use in the United States in May 2022, the European Union in September 2022, Canada in November 2022, and Australia in December 2022. The U.S. Food and Drug Administration (FDA) considers the tirzepatide as the first drug.

For effective administration of the tirzepatide alone, development of a technology to ensure a long-term effective pharmacological effect is required. Accordingly, the inventor of the present invention devises a technique for encapsulating the tirzepatide in a microsphere that is formed of a biodegradable polymer for a long-term release thereof. However, in this case, since a bioavailability of the tirzepatide encapsulated in the microsphere is low or a content of a drug included in the microsphere is low, a large amount of the microsphere needs to be administered to exhibit the long-term effective pharmacological effect. However, when administering a large amount of the microsphere in vivo, there is a problem that it is difficult for a patient to administer by oneself (self-administer) due to the difficulty of subcutaneous injection and the possibility of that pain and inflammatory response at an administration site are also very high.

### [Disclosure]

### [Technical Problem]

The present invention is devised to solve the problems as described above and is directed to providing a pharmaceutical composition including a sustained-release microsphere that includes tirzepatide or a pharmaceutically acceptable salt thereof with a high content thereof, a biodegradable polymer, and an initial burst inhibiting agent that exhibit a long-term stable drug release, and a method of preparing the sustained-release microsphere.

### [Technical Solution]

Hereinafter, the present invention will be described in detail.

As used herein, the term "one or more types" refers to a "number" corresponding to one or more. In the present invention, when there are one or more types of configurations, it may preferably be one type, two or more types, three or more types, one to three types, or one to two types, but it is not limited thereto. The term "one or more types" may be used interchangeably with the term "at least one" in the present invention.

To achieve the above purpose,
as an aspect of the present invention, a pharmaceutical composition including a biodegradable polymer and a tirzepatide sustained-release microsphere, wherein an amount of the tirzepatide is 8 wt.% or more of the tirzepatide based on the total weight of the microsphere and an amount of an initial burst inhibiting agent is 5 ppm to 2000 ppm relative to a weight of the tirzepatide is provided.

As another aspect, the biodegradable polymer may be at least one selected from a group consisting of: a polymer selected from a group consisting of block copolymers of polylactide (PLA), polyglycolide (PGA), polylactide-co-glycolide (PLGA), polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and block copolymers of poly(butylene succinate lactide) (PBSLA); a simple mixture of two or more types selected from said polymer; a copolymer with said selected polymer and polyethylenglycol (PEG); and a polymer-sugar complex in which said polymer or the copolymer and sugar are bonded.

As still another aspect, a pharmaceutically acceptable salt of the tirzepatide may be sodium salt, acetate, benzoate, hydroxynaphthoate, napadisylate, or pamoate of the tirzepatide.

As still another aspect, an intrinsic viscosity of the biodegradable polymer may be 0.16 to 1.7 dL/g. More specifically, the intrinsic viscosity of polylactide-co-glycolide (PLGA) or polylactide (PLA) among the biodegradable polymers may be 0.16 to 1.7 dL/g.

As still another aspect, an average particle size of the microsphere including the tirzepatide or the pharmaceutically acceptable salt thereof and the initial burst inhibiting agents may be 5 µm to 100 µm.

As still another aspect, a span value of the microsphere including the tirzepatide or the pharmaceutically acceptable salt thereof and the initial burst inhibiting agent may be 1.5 or less.

As still another aspect, a total weight of the microsphere including the tirzepatide or the pharmaceutically acceptable salt thereof and the initial burst inhibiting agent included in the pharmaceutical composition may be 20 to 1000 mg, 20 to 800 mg, 20 to 600 mg, 20 to 400 mg, 20 to 200 mg, 20 to 100 mg, 30 to 1000 mg, 30 to 800 mg, 30 to 600 mg, 30 to 400 mg, 30 to 200 mg, 30 to 100 mg, 40 to 1000 mg, 40 to 800 mg, 40 to 600 mg, 40 to 400 mg, 40 to 200 mg, 40 mg to 100 mg, 50 mg to 1000 mg, 50 mg to 800 mg, 50 mg to 600 mg, 50 mg to 400 mg, 50 mg to 200 mg, or 50 mg to 100 mg.

As still another aspect, the microsphere may further include one or more types of release control agents selected from a group consisting of butyric acid, valeric acid, caproic acid, enantic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, behenic acid, arachidic acid, isocrotonic acid, oleic acid, elaidic acid, sorbic acid, linoleic acid, arachidonic acid, benzoic acid, hydroxynaphthoic acid, napadisylic acid, naphthalenesulfonic acid, and pamoic acid.

As still another aspect, the microsphere may include 10 to 500 mg/kg of Na.

As still another aspect, the microsphere may include 1 to 100 mg/kg of P.

As still another aspect, the present invention provides a method of preparing a sustained-release microsphere including tirzepatide or a pharmaceutically acceptable salt thereof and an initial burst inhibiting agent.

As still another aspect, the method of preparing may be using a continuous phase including the initial burst inhibiting agent.

As still another aspect, any substance that may set a pH of the continuous phase to 7 or more when dissolving in the continuous phase may be used as the initial burst inhibiting agent.

As still another aspect, the initial burst inhibiting agent may use one or more types of substances selected from a group consisting of phosphate salt, hydroxide salt, phosphide salt, phosphite salt, carbonate salt, bicarbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt, and nitride salt of an alkali metal, an alkaline-earth metal, or ammonium. As still another aspect, the initial burst inhibiting agent may be at least one selected from a group consisting of disodium phosphate, dipotassium phosphate, and diammonium phosphate. As yet another aspect, the present invention provides a pharmaceutical composition including a biodegradable polymer and a sustained-release microsphere, wherein a content of tirzepatide is 8 wt.% or more of the tirzepatide based on the total weight of the microsphere and an initial drug burst rate is 10% or less, and a method of preparing the same.

### [Advantageous Effects]

According to a preparation example of the present invention, a sustained-release pharmaceutical composition including tirzepatide or a pharmaceutically acceptable salt thereof and an initial burst inhibiting agent has an effect of minimizing pain of a patient and a side effect at an administration site that may occur during administration because not only a dosage of a microsphere is reduced due to a high content and a high bioavailability is exhibited as a rapid initial burst is inhibited by the initial burst inhibiting agent despite including a high content of the tirzepatide, but also the dosage of the microsphere is reduced while having high bioavailability and exhibiting a long-acting drug efficacy for one month or more when administered in vivo.

### [Description of Drawings]

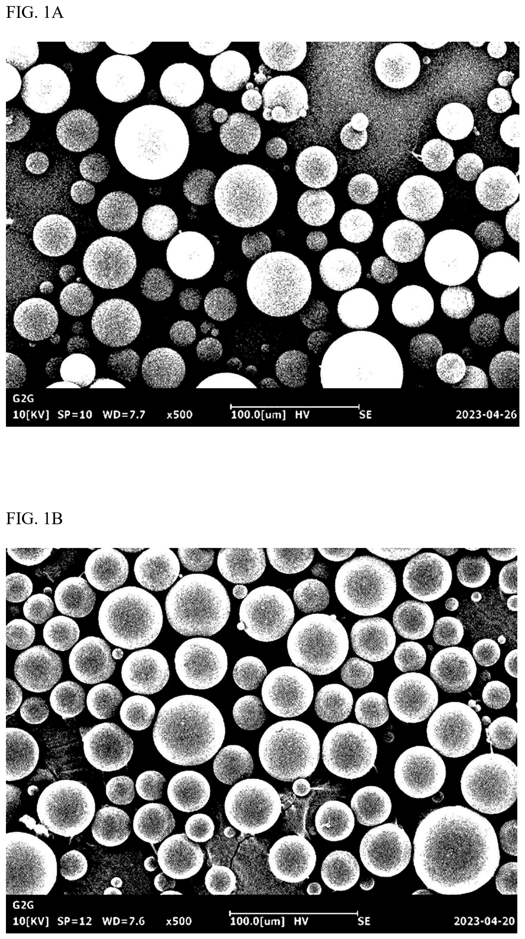

FIGS. 1A and B are scanning electron microscope photographs confirming morphological features of a microsphere manufactured according to Example 1 and Comparative Example 1, respectively.

### [Detailed Description]

Hereinafter, the present invention will be described in detail.

The present invention includes tirzepatide or a pharmaceutically acceptable salt thereof as an active ingredient.

Tirzepatide is an acylated peptide engineered to activate GIP and GLP-1 receptors, which are key mediators of insulin secretion that are also expressed in a brain region that regulates food consumption. The tirzepatide is designed to be administered subcutaneously once a week.

A structure of the tirzepatide is as shown in Chemical Formula 1 below.

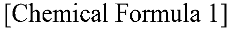

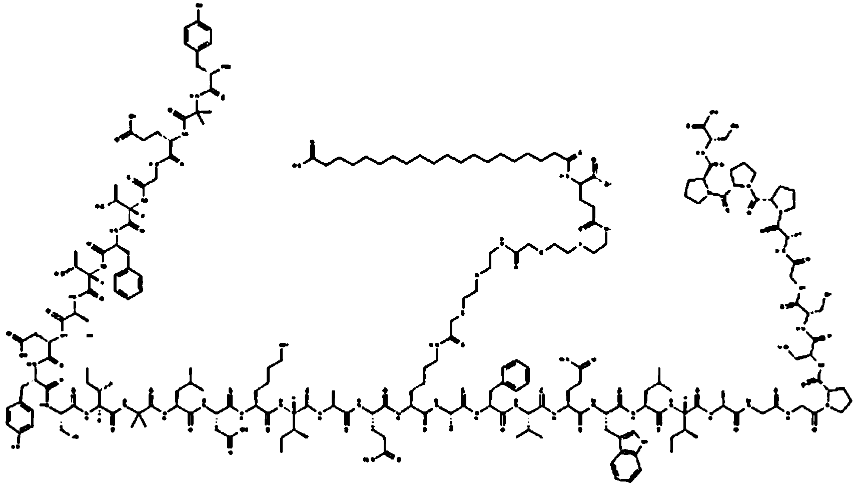

The tirzepatide may exist in a form of a salt, particularly in a form of a pharmaceutically acceptable salt. Any salt commonly used in the art may be used as the salt without limitation. As used herein, the term "pharmaceutically acceptable salt" refers to any organic or inorganic addition salt of the compound of which side effects caused by the salt do not reduce the beneficial effects of the active ingredient by a concentration that is relatively non-toxic and harmless to the patient. A specific example includes sodium salt, acetate, benzoate, hydroxynaphthoate, napadisylate, or pamoate of the tirzepatide, but it is not limited thereto.

The tirzepatide or the pharmaceutically acceptable salt thereof as the active ingredient of the present invention may be in various forms, for example, in an amorphous form or a crystalline form.

The pharmaceutical composition according to the present invention may have an area under a blood concentration-time curve (AUC₀₋₂₄ₕᵣ) of the tirzepatide up to 24 hours after administration of 20% or less, 10% or less, 5% or less, 0.1 to 20%, 1 to 10%, or 1 to 5% relative to a total area under the blood concentration-time curve (AUCₜₒₜₐₗ).

The pharmaceutical composition according to the present invention may have an area under the blood concentration-time curve (AUC_{0-QXM}) of the tirzepatide up to an administration interval date after administration of 70% or less, 65% or less, 60% or less, 20 to 70%, 20 to 65%, 20 to 60%, 25 to 70%, 25 to 65%, 25 to 60%, 30 to 70%, 30 to 65% or 30 to 60% in a case of a human, and 95% or less, 93% or less, 90% or less, 50 to 95%, 50 to 93%, 50 to 90%, 55 to 95%, 55 to 93%, 55 to 90%, 60 to 95%, 60 to 93%, or 60 to 90% in a case of rats relative to the total area under the blood concentration-time curve (AUCₜₒₜₐₗ).

In addition, the pharmaceutical composition according to the present invention may be a pharmaceutical composition including a sustained-release microsphere in which a content of the tirzepatide is 8 wt.% or more of the tirzepatide relative to a total weight of the microsphere and an initial burst of the drug is less than 10% for 24 hours.

A biodegradable polymer included in a tirzepatide sustained-release microsphere that is included in the pharmaceutical composition according to the present invention, for example, is selected from a group consisting of a polymer selected from a group consisting of polylactide (PLA), polyglycolide (PGA), polylactide-co-glycolide (PLGA), polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly(butylene succinate lactide) (PBSLA), a simple mixture of two or more types selected from said polymer, a copolymer with said polymer and polyethylenglycol (PEG), and a polymer-sugar complex in which said polymer or the copolymer and sugar are bonded, but it is not limited thereto.

In addition, that the biodegradable polymer is a simple mixture of two or more (i.e., a simple mixture including two or more of said selected polymers) may include two or more types of different types of polymers among the polymers exemplified non-restrictively, or may be a combination or blend of the same type of the polymers, but may be a combination of the polymers having different intrinsic viscosities and/or monomer ratios (e.g., a combination or blend of two or more poly (lactide-co-glycolide) having different intrinsic viscosities), or may be a same type of the polymers that differ in terminal groups (e.g., a same type of the polymers with an ester terminal group or an acid terminal group).

As a specific aspect, the pharmaceutical composition according to the present invention may include a microsphere including two or more types of the biodegradable polymers. As another specific embodiment, the pharmaceutical composition according to the present invention may include two or more types of microspheres each including at least one of two or more types of polymers selected from the biodegradable polymers.

The biodegradable polymer may be a biodegradable polymer having an intrinsic viscosity of 0.16 to 1.7 dL/g, 0.2 to 1.3 dL/g, or 0.24 to 1.2 dL/g, in consideration of factors such as release properties and a manufacturing process of a drug. The intrinsic viscosity refers to a viscosity measured at 0.1% (w/v) concentration in chloroform at 25 °C using an Ubbelohde viscometer.

In a case of the polylactide-co-glycolide, a molar ratio of lactide to glycolide in the copolymer may be 40:60 to 90:10, 45:55 to 85:15, or 50:50 to 75:25, for example, 45:55, 50:50, 75:25, or 85:15.

When the intrinsic viscosity of the biodegradable polymer is less than 0.16 dL/g, a molecular weight of the polymer is insufficient, and thus it is difficult to exhibit a sustained-release effect of the tirzepatide or the pharmaceutically acceptable salt thereof, and when the intrinsic viscosity of the biodegradable polymer exceeds 1.7 dL/g, release of the tirzepatide or the pharmaceutically acceptable salt thereof may be excessively delayed. In addition, when manufacturing the microsphere using a polymer with high intrinsic viscosity, there is a problem that an excessive amount of a manufacturing solvent is used due to a high viscosity of the polymer, and thus it is difficult to manufacture a reproducible microsphere. Examples of a commercially available polymer having the above-described properties include RG502H, RG503H, RG504H, RG502, RG503, RG504, RG653H, RG752H, RG753H, RG752S, RG755S, RG750S, RG757S, RG858S, R202H, R203H, R205H, R202S, R203S, R205S, R206S, and R207S of Resomer^{®} series of Evonik, and PDL 02A, PDL 02, PDL 04, PDL 05, PDLG 7502A, PDLG 7502, PDLG 7507, PDLG 5002A, PDLG 5002, PDLG 5004A, and PDLG 5004 of Corbion.

An amount of the biodegradable polymer in the sustained-release microsphere including the tirzepatide or the pharmaceutically acceptable salt thereof and an initial burst inhibiting agent according to the present invention may select 60 wt.% or more, 62 wt.% or more, 65 wt.% or more, 67 wt.% or more, or 70 wt.% or more as an upper limit, and may select 92 wt.% or less, 91 wt.% or less, 90 wt.% or less, 89 wt.% or less, or 88 wt.% or less as a lower limit based on the total weight of the microsphere, or may be included in a range consisting of a combination of the upper limit and the lower limit. For example, the range may be 60 wt.% to 92 wt.%, 65 wt.% to 90 wt.%, or 70 wt.% to 88 wt.%, but it is not limited thereto.

An amount of the tirzepatide or the pharmaceutically acceptable salt thereof in the sustained-release microsphere including the tirzepatide or the pharmaceutically acceptable salt thereof and the initial burst inhibiting agent according to the present invention is preferably 8 wt.% or more, 12 wt.% or more, 14 wt.% or more, 37 wt.% or less, 35 wt.% or less, or 33 wt.% or less of the tirzepatide relative to the total weight of the microsphere. When the content of the tirzepatide or the pharmaceutically acceptable salt thereof in the microsphere is less than 8 wt.% based on the tirzepatide, an amount of the polymer used based on the drug may be excessively high, and thus the bioavailability of the tirzepatide or the pharmaceutically acceptable salt thereof may decrease, and when the content is excessively high, the initial burst of the tirzepatide or the pharmaceutically acceptable salt thereof may increase, and thus it may not be preferable.

The pharmaceutical composition including the sustained-release microsphere according to the present invention may include the initial burst inhibiting agent in the microsphere in less than a certain amount.

The initial burst inhibiting agent in the present invention is a substance included in a continuous phase to inhibit a rapid release of the active ingredient in a process of manufacturing the microsphere, and it may exist in the microsphere according to the present invention in less than a certain amount. The continuous phase refers to a substance that enables a dispersed phase including an active ingredient such as the tirzepatide and the biodegradable polymer to be dispersed when an emulsion and solvent extraction evaporation method is used in the method of manufacturing the microsphere, and may be an aqueous solution including a surfactant in the present invention, but it is not limited thereto. The initial burst inhibiting agent according to the present invention is included in the continuous phase that is used in such a method of manufacturing, and accordingly, it may be included in the microsphere according to the present invention in a certain amount after manufacturing. Matters concerning the continuous phase may be applied in the same manner as those described in the method of preparing the microsphere according to the present invention described below.

As a specific aspect, the initial burst inhibiting agent may be included in 5 ppm or more, 10 ppm or more, 20 ppm or more, 30 ppm or more, 40 ppm or more, 50 ppm or more, 100 ppm or more, 150 ppm or more, 200 ppm or more, 250 ppm or more, 200 ppm or more, 300 ppm or more, 350 ppm or more, 400 ppm or more, 450 ppm or more, 500 ppm or more, 550 ppm or more, 600 ppm or more, 650 ppm or more, 700 ppm or more, 750 ppm or more, 800 ppm or more, 850 ppm or more, 900 ppm or more, 950 ppm or more, 1000 ppm or more, 1100 ppm or more, 1200 ppm or more, 1300 ppm or more, 1400 ppm or more, or 1500 ppm or more as an upper limit, and 2000 ppm or less, 1900 ppm or less, 1800 ppm or less, 1700 ppm or less, 1600 ppm or less, 1500 ppm or less, 1400 ppm or less, 1300 ppm or less, 1200 ppm or less, 1100 ppm or less, 1000 ppm or less, 900 ppm or less, 800 ppm or less, 700 ppm or less, 600 ppm or less, or 500 ppm or less as a lower limit based on the total weight of the microsphere, and may be included in a range consisting of as a combination of the upper limit and the lower limit. For example, the initial burst inhibiting agent may be included in 5 ppm to 2000 ppm, preferably 10 to 1500 ppm, more preferably 20 to 1000 ppm, and most preferably 20 to 500 ppm based on the total weight of the microsphere.

As a specific aspect, any substance that enables the pH of the continuous phase to be set to 7 or more when dissolving in the continuous phase may be used as the initial burst inhibiting agent. The initial burst inhibiting agent may be at least one selected from a group consisting of phosphate salt, hydroxide salt, phosphide salt, phosphite salt, carbonate salt, bicarbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt, and nitride salt of an alkali metal, an alkaline-earth metal, and ammonium, but it is not limited thereto.

As a specific aspect, the initial burst inhibiting agent may be at least one selected from a group consisting of phosphate salt of two or more alkali metals, carbonate salt of one or more alkali metals, and phosphite salt of ammonium, but it is not limited thereto.

As a more specific aspect, the phosphate salt of the two or more alkali metals refer to a phosphate salt including two or more alkali metal ions, for example, disodium phosphate (Na₂HPO₄) or dipotassium phosphate (K₂HPO₄), bicarbonate salt of the one or more alkali metals refer to bicarbonate salt including one or more alkali metal ions, for example, sodium bicarbonate (NaHCO₃), the carbonate salt the two or more alkali metals refer to carbonate salt including two or more alkali metal ions, for example, sodium carbonate (Na₂CO₃), and the phosphate salt of the two or more ammoniums refers to phosphate salt including two or more ammonium ions, for example, diammonium phosphate ((NH₄)₂HPO₄), and it is possible to use alone or by mixing two or more types among them.

It is preferable for the microsphere including the tirzepatide or the pharmaceutically acceptable salt thereof and the initial burst inhibiting agent according to the present invention to have a uniform particle distribution of an average particle size of 5 µm to 100 µm, 5 µm to 90 µm, 5 µm to 80 µm, 10 µm to 90 µm, 10 µm to 80 µm, 15 µm to 100 µm, 15 µm to 90 µm, 15 µm to 80 µm, 70 µm to 100 µm, 70 µm to 90 µm, 70 µm to 80 µm, 60 µm to 100 µm, 60 µm to 80 µm, 60 µm to 70 µm, 20 µm to 90 µm, 20 µm to 70 µm, 20 µm to 60 µm, 30 µm to 80 µm, 30 µm to 60 µm, 40 µm to 70 µm, 40 µm to 50 µm, 30 µm to 40 µm, 20 µm to 30 µm, 5 µm to 30 µm, 5 µm to 20 µm, 10 µm to 20 µm, or 5 µm to 10 µm. The term "average particle size" used in the present invention refers to a particle size corresponding to 50% of the volume % in a particle size distribution curve, which refers to a median diameter and is represented as D50 or D(v, 0.5).

When the average particle size of the microsphere including the tirzepatide or the pharmaceutically acceptable salt thereof and the initial burst inhibiting agent is less than 5 µm, the release of the tirzepatide or the pharmaceutically acceptable salt drug thereof from the microsphere may be excessively fast, and thus it may not be preferable. When the average particle size exceeds 100 µm, an injection needle may become too thick when administering to a human body, thereby causing pain during injection or the drug may leak out from an injection site after injection, and thus it may not be preferable.

It is preferable for the microsphere including the tirzepatide or the pharmaceutically acceptable salt thereof and the initial burst inhibiting agent of the present invention to have a uniform particle distribution. The microsphere including the tirzepatide or the pharmaceutically acceptable salt thereof and the initial burst inhibiting agent having a uniform particle distribution has a smaller deviation during injection and may be administered in a more accurate amount compared to a non-uniform microsphere. It is preferable for a span value of the microsphere including the tirzepatide or the pharmaceutically acceptable salt thereof and the initial burst inhibiting agent of the present invention to be 1.5 or less. More preferably, it is preferable for the span value to be 1.2 or less. More specifically, the span value may be 1.5 or less, 1.2 or less, 0.1 to 1.5, 0.3 to 1.5, 0.5 to 1.5, 0.1 to 1.0, 0.4 to 1.0, 0.6 to 1.0, 0.2 to 0.8, or 0.4 to 0.8. The term "span value" used in the present invention is an index of the uniformity of the particle size of the microsphere and refers to a value obtained by a formula of Span value=(Dv0.9-Dv0.1)/Dv0.5. Here, Dv0.1 refers to a particle size corresponding to 10% of the volume% in the particle size distribution curve of the microsphere, Dv0.5 refers to a particle size corresponding to 50% of the volume% in the particle size distribution curve of the microsphere, and Dv0.9 refers to a particle size corresponding to 90% of the volume% in the particle size distribution curve of the microsphere.

The sustained-release microsphere including the tirzepatide or the pharmaceutically acceptable salt thereof and the initial burst inhibiting agent of the present invention is administered by an injection route, for example, subcutaneous injection and is especially self-administered, and thus it is preferable that the tirzepatide is released over a relatively long period of time. Preferably, the sustained-release microsphere in the pharmaceutical composition according to the present invention may release the tirzepatide or the pharmaceutically acceptable salt thereof for 1 month or more, 2 months or more, 3 months or more, 1 month to 2 months, 1 month to 3 months, 1 month to 4 months, 1 month to 5 months, 1 month to 6 months, 2 months to 6 months, 2 months to 5 months, 2 months to 4 months, 2 months to 3 months, 3 months to 5 months, or 3 months to 4 months, but it is not limited thereto. In addition, the sustained-release microsphere including the tirzepatide or the pharmaceutically acceptable salt thereof and the initial burst inhibiting agent of the present invention is not particularly limited in the release pattern, but it is preferable that the tirzepatide or the pharmaceutically acceptable salt thereof is released for 24 hours when administered in vivo at a release rate (release amount) of less than 10%, less than 15%, or less than 20% as an upper limit, or at a release rate of 0.001 % or more, 0.01% or more, 0.1% or more, or 1% or more as a lower limit, or at a release rate in a range of a combination of the upper limit and the lower limit.

In addition, a total amount (total weight) of the sustained-release microsphere formulation including the tirzepatide or the pharmaceutically acceptable salt thereof and the initial burst inhibiting agent in the pharmaceutical composition of the present invention may be 30 to 3000 mg, 30 to 2500 mg, 30 to 2000 mg, 30 to 1500 mg, 30 to 1250 mg, 30 to 1000 mg, 60 to 3000 mg, 60 to 2500 mg, 60 to 2000 mg, 60 to 1500 mg, 60 to 1250 mg, 100 to 3000 mg, 100 to 2500 mg, 100 to 2000 mg, 100 to 1500 mg, 200 to 3000 mg, 200 to 2500 mg, 200 to 1500 mg, 400 to 3000 mg, 400 to 2500 mg, 400 to 2000 mg, or 400 to 1500 mg. As the sustained-release microsphere including the tirzepatide or the pharmaceutically acceptable salt thereof and the initial burst inhibiting agent are included in the composition in the above range, the composition according to the present invention has an advantage of minimizing an inflammatory response at a site of administration and enabling self-administration by the patient.

The microsphere including the tirzepatide or the pharmaceutically acceptable salt thereof and the initial burst inhibiting agent included in the composition of the present invention may further include a release control agent. An example of a substance used as the release control agent may be at least one selected from butyric acid, valeric acid, caproic acid, enantic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, behenic acid, arachidic acid, isocrotonic acid, oleic acid, elaidic acid, sorbic acid, linoleic acid, arachidonic acid, benzoic acid, hydroxynaphthoic acid, napadisylic acid, naphthalenesulfonic acid, and pamoic acid, but it is not limited thereto. Preferably, the release control agent may be hydroxynaphthoic acid, napadisylic acid, or pamoic acid, but it is not limited thereto.

The pharmaceutical composition including the microsphere that includes the tirzepatide or the pharmaceutically acceptable salt thereof and the initial burst inhibiting agent according to the present invention may be formulated into various forms of formulations and for example, it may be a dosage form of a known parenteral administration formulation. Accordingly, the pharmaceutical composition according to the present invention may further include a thickening agent, a stabilizing agent, an isotonic agent, a surfactant, an excipient and/or a carrier in addition to the tirzepatide or the pharmaceutically acceptable salt thereof and the initial burst inhibiting agent. An available isotonic agent may be a water-soluble excipient or sugar such as mannitol, sucrose, sorbitol, trehalose, lactose, sodium chloride, etc., and examples of the thickening agent may include carmellose sodium, carboxymethyl cellulose sodium, povidone, etc. In addition, sodium dihydrogen phosphate, anhydrous citric acid, sodium hydroxide, sodium chloride, etc., may be used as the stabilizing agent.

The pharmaceutical composition according to the present invention may be administered in a therapeutically effective amount of the tirzepatide, for example, an effective amount for treating diabetes, specifically type 2 diabetes, beta-cell dysfunction, hypertension, hyperlipidemia, obesity, non-alcoholic steatohepatitis, or degenerative neurological diseases such as Alzheimer's disease and Parkinson's disease. The therapeutically effective amount of the tirzepatide may be assessed by a physician. The pharmaceutical composition according to the present invention including the tirzepatide may be administered once a month or once a quarter. In some specific examples, a monthly dosage of the composition according to the present invention may be 5 mg to 500 mg, 5 mg to 400 mg, 5 mg to 300 mg, 5 mg to 150 mg, 5 mg to 100 mg, 5 mg to 50 mg, 5 mg to 40 mg, 5 mg to 30 mg, 10 mg to 500 mg, 10 mg to 400 mg, 10 mg to 300 mg, 10 mg to 150 mg, 10 mg to 100 mg, 10 mg to 50 mg, 10 mg to 40 mg, 10 mg to 30 mg, 20 mg to 500 mg, 20 mg to 400 mg, 20 mg to 300 mg, 20 mg to 150 mg, 20 mg to 100 mg, 40 mg to 500 mg, 40 mg to 400 mg, 40 mg to 300 mg, 40 mg to 150 mg, 50 mg to 500 mg, 50 mg to 400 mg, 50 mg to 300 mg, 50 mg to 150 mg, 100 mg to 500 mg, 100 mg to 400 mg, 100 mg to 300 mg, or 100 mg to 150 mg based on the tirzepatide. The pharmaceutical composition according to the present invention including the microsphere that includes the tirzepatide or the pharmaceutically acceptable salt thereof and the initial burst inhibiting agent according to the present invention may also be administered parenterally, for example, by subcutaneous injection. The pharmaceutical composition according to the present invention may be composed of a drug phase including the microsphere that includes the tirzepatide or the pharmaceutically acceptable salt thereof and the initial burst inhibiting agent and a solvent phase used to suspend the microsphere, and may be in a form of a dual chamber syringe having the drug phase in one chamber and the solvent phase in the other chamber, or in a form of a prefilled syringe in which the drug phase is suspended in the solvent phase. When composed in the prefilled syringe form in which the drug phase is suspended in the solvent phase in a prefilled syringe, the solvent phase used may be an injectable oil including a medium-chain oil, mineral oil, etc.

In a specific embodiment, the sustained-release microsphere including the tirzepatide or the pharmaceutically acceptable salt thereof and the initial burst inhibiting agent included in the pharmaceutical composition according to the present invention has a high drug content based on the content of the microsphere, and at the same time, inhibits an excessive initial burst of the drug that may cause fatal side effects and exhibits sufficient drug efficacy as a GIP analogue for a desired period due to the high bioavailability, and thus it is useful for the prevention or treatment of diabetes, specifically type 2 diabetes, beta-cell dysfunction, hypertension, hyperlipidemia, obesity, non-alcoholic steatohepatitis, Alzheimer's disease, or Parkinson's disease.

As another aspect, the present invention provides a method of preparing the sustained-release microspheres including the tirzepatide or the pharmaceutically acceptable salt thereof and the initial burst inhibiting agent.

As another aspect, the present invention provides a method of preparing the sustained-release microsphere having a significantly inhibited initial burst despite including a high content of the tirzepatide or the pharmaceutically acceptable salt thereof.

Hereinafter, a method of preparing a sustained-release microsphere injection including the tirzepatide or the pharmaceutically acceptable salt thereof and the initial burst inhibiting agent of the present invention will be described in detail.

The sustained-release microsphere injection including the tirzepatide or the pharmaceutically acceptable salt thereof and the initial burst inhibiting agent according to the present invention may be manufactured, for example, by using "emulsion solvent extraction and evaporation method", but the method of preparing is not limited thereto.

As an aspect of a specific method of preparing, the present invention provides a method of manufacturing the sustained-release microsphere with an oil-in-water (O/W) emulsion including the following steps:
(a) preparing an oil phase (O phase) as a dispersed phase obtained by dissolving the tirzepatide or the pharmaceutically acceptable salt thereof and one or more types of biodegradable polymers in an organic solvent;
(b) preparing a continuous phase (W phase) by adding an initial burst inhibiting agent to an aqueous solution including a surfactant and adding the dispersed phase of the step (a) to obtain a dispersed phase in an emulsion state;
(c) extracting and evaporating an organic solvent from the dispersed phase in the emulsion state obtained in the step (b) in the continuous phase (W phase) to form a microsphere; and
(d) recovering the microsphere.

As another aspect, the present invention provides a method of preparing the sustained-release microsphere with a water-in-oil-in-water (W/O/W) emulsion including the following steps:
(a') preparing an aqueous phase (W1 phase) by dissolving the tirzepatide or the pharmaceutically acceptable salt thereof in an aqueous solution and preparing an oil phase (O phase) by dissolving one or more types of biodegradable polymers in an organic solvent to prepare a primary W1/O emulsion as a dispersed phase in which the aqueous phase (W1 phase) and the oil phase (O phase) are mixed;
(b') preparing a continuous phase (W2 phase) by adding an initial burst inhibiting agent to an aqueous solution including a surfactant and adding the dispersed phase obtained in the step (a') to obtain a dispersed phase in a secondary W1/O/W2 emulsion state;
(c') extracting and/or evaporating an organic solvent from the dispersed phase in the secondary W1/O/W2 emulsion state obtained in the step (b') in the continuous phase (W2 phase) to form a microsphere; and
(d') recovering the microsphere.

As still another aspect, a pH of the continuous phase used in the methods of preparing may be 7 or more.

In the preparing of the sustained-release microsphere including the tirzepatide or the pharmaceutically acceptable salt thereof and the initial burst inhibiting agent according to the present invention, the sustained-release microsphere includes a high content of the tirzepatide the pharmaceutically acceptable salt thereof relative to the weight of the microsphere, and at the same time, an excessive initial burst of the tirzepatide or the pharmaceutically acceptable salt thereof is inhibited, the bioavailability is high, and the following biodegradable polymers may be used to release at a constant concentration for a desired long period of time, for example, 1 month or more, 3 months or more, 1 month to 2 months, 1 month to 3 months, 1 month to 4 months, 1 month to 5 months, 1 month to 6 months, 2 months to 6 months, 2 months to 5 months, 2 months to 4 months, 2 months to 3 months, 3 months to 5 months, or 3 months to 4 months, but it is not limited thereto. Specifically, it is preferable to use one or more polymers, preferably two or more types of polymers selected from a group consisting of a polymer selected from a group consisting of polylactide (PLA), polyglycolide (PGA), polylactide-co-glycolide (PLGA), polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly(butylene succinate lactide) (PBSLA), a simple mixture of two or more types selected from said polymer, a copolymer with said polymer and polyethylenglycol (PEG), and a polymer-sugar complex in which the polymer or the copolymer and sugar are bonded. As a specific embodiment, in the method of manufacturing according to the present invention, poly (lactide-co-glycolide) and/or a polylactide (PLA) polymer may be used as the biodegradable polymer.

In the present invention, two or more different biodegradable polymers may include two or more polymers having different repeating units constituting the polymers and two or more polymers having different molar ratios of the repeating units when including two or more repeating units. As an example, the microsphere may be a mixture of a microsphere including poly-lactide-co-glycolide and a microsphere including a polylactide (PLA) polymer, or a microsphere including both the poly-lactide-co-glycolide and the polylactide (PLA) polymer.

In addition, as a specific example, when there are two types of the biodegradable polymers that are different from each other, a content ratio of the biodegradable polymers may be a weight ratio of 0.5:10 to 10:0.5, 0.5:8 to 8:0.5, 1:10 to 10:1, 1:4 to 4:1, 1:3 to 3:1, or 1:2 to 2:1, but it is not limited thereto.

In addition, unless otherwise defined, the description of the biodegradable polymer described in the microsphere according to the present invention may be applied as it is.

As a more specific aspect, when polylactide-co-glycolide is used as two or more types of biodegradable polymers to manufacture the sustained-release microsphere including the tirzepatide or the pharmaceutically acceptable salt thereof and the initial burst inhibiting agent according to the present invention, at least one biodegradable polymer having an intrinsic viscosity of 0.16 dL/g to 0.45 dL/g may be included.

In the case of the polylactide-co-glycolide, a molar ratio of lactide to glycolide in the copolymer may be 40:60 to 90:10, 45:55 to 85:15, or 50:50 to 75:25, for example, 45:55, 50:50, 75:25, or 85:15.

In addition, unless otherwise defined, the description of the biodegradable polymer described in the microsphere according to the present invention may be applied in the same manner.

The organic solvent used to dissolve at least one biodegradable polymer in the step (a) or (a') of the specific method of preparing is one or more organic solvents. In addition, a mixed organic solvent in which two or more types of organic solvents are mixed may be used as the organic solvent. As a specific aspect, the mixed solvent may be a mixed solvent of an organic solvent that is miscible with water and an organic solvent that is not miscible with water. In this case, it is preferable to use an organic solvent with a water-immiscible property in 50% (v/v) or more, 60% (v/v) or more, 50 to 99.9% (v/v), 50 to 90% (v/v), 50 to 80% (v/v), 50 to 70% (v/v), 60 to 90% (v/v), or 60 to 80% (v/v). By using the water-immiscible property of the organic solvent, the dispersed phase may be homogeneously mixed in the continuous phase including the surfactant in the step (b) or (b') described later to form the emulsion. A type of the organic solvent that dissolves one or more of the biodegradable polymers is not particularly limited, but preferably a mixed solvent of one or more solvents selected from a group consisting of dichloromethane (DCM), chloroform, ethyl acetate, methyl ethyl ketone, acetone, acetonitrile, dimethyl sulfoxide, dimethyl formamide, n-methyl pyrrolidone, acetic acid, methyl alcohol, ethyl alcohol, propyl alcohol and benzyl alcohol, more preferably one type of solvent selected from dichloromethane (DCM) and ethyl acetate, and one or more types of organic solvents selected from dimethyl sulfoxide, n-methyl pyrrolidone, methyl alcohol, and acetic acid may be used. In an embodiment, dichloromethane (DCM) and acetic acid (glacial acetic acid) may be used by mixing as the organic solvent.

The method for homogeneously mixing the continuous phase including the dispersed phase and the surfactant in the step (b) or (b') is not particularly limited, but it may be performed using a high-speed stirrer, an inline mixer, a membrane emulsion method, a microfluidics emulsion method, an ultrasonic mixer, or a static mixer, alone or by using two or more types. When forming an emulsion using the high-speed stirrer, the inline mixer, the ultrasonic mixer, or the static mixer, it is difficult to obtain a uniform emulsion, and thus it is preferable to additionally perform a sieving process, etc. between steps (c) and (d) or between steps (c') and (d') described later.

The type of the surfactant used in the step (b) or (b') is not particularly limited, and any type that may help form a dispersed phase with a stable droplet inside the continuous phase may be used. As the surfactant, polyvinyl alcohol, methyl-cellulose, polyvinylpyrrolidone, carboxymethylcellulose, lecithin, gelatin, polyoxyethylene sorbitan fatty acid ester, polyoxyethylene castor oil derivative, etc., may be used alone or by mixing two or more types.

In the step (b) or (b'), a content of the surfactant in the continuous phase including the surfactant may be 0.01 w/v% to 20 w/v%, preferably 0.03 w/v% to 18 w/v%, 0.05 w/v% to 15 w/v%, 0.07 w/v% to 10 w/v%, or 0.1 w/v% to 5 w/v%, based on a total volume of the continuous phase including the surfactant. When the content of the surfactant is less than 0.01 w/v%, a droplet-shaped dispersed phase or the emulsion may not be formed inside the continuous phase, and when the content of the surfactant exceeds 20 w/v%, it may be difficult to remove the surfactant after fine particles are formed inside the continuous phase due to the excessive amount of the surfactant.

An aqueous solution including the surfactant and the initial burst inhibiting agent may be used as the continuous phase used in the step (b) or (b'). In addition, the continuous phase may also be used by adding to the aqueous solution including the surfactant so as to reach a final concentration of the initial burst inhibiting agent.

As the initial burst inhibiting agent is used, the initial burst of the microsphere enclosed with a high content of the drug may be controlled, and a substance capable of setting the pH of the continuous phase to 7.0 or more may be used as the initial burst inhibiting agent.

The type of the initial burst inhibiting agent is not particularly limited, and any basic salt that may be dissolved in the continuous phase and maintains a pH of 7.0 or more may be used. The initial burst inhibiting agent may use one or more types of substances selected from a group consisting of phosphate salt, hydroxide salt, phosphide salt, phosphite salt, carbonate salt, bicarbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt, and nitride salt of an alkali metal, an alkaline-earth metal, and ammonium.

As a specific aspect, the initial burst inhibiting agent may be at least one selected from a group consisting of phosphate salts of two or more alkali metals, carbonate salts of one or more alkali metals, bicarbonate salts of one or more alkali metals, and phosphate salts of two or more ammoniums, but it is not limited thereto.

As a more specific aspect, the phosphate salt of the two or more alkali metals is disodium phosphate (Na₂HPO₄) or dipotassium phosphate (K₂HPO₄), the carbonate salt of the one or more alkali metals is sodium bicarbonate (NaHCO₃) or sodium carbonate (Na₂CO₃), and the phosphate salt of the two or more ammoniums is diammonium phosphate ((NH₄)₂HPO₄), and it is possible to use alone or by mixing two or more types among them.

In the continuous phase, the final concentration of the initial burst inhibiting agent may be 0.1 to 5.0 (w/v)%, preferably 0.1 to 4.0 (w/v)%, more preferably 0.2 to 4.0 (w/v)%, still more preferably 0.3 to 3.5 (w/v)%, particularly preferably 0.4 to 3.0 (w/v)%, and particularly more preferably 0.5 to 2.5 (w/v)%.

When a content of the initial burst inhibiting agent exceeds 5.0 w/v%, a phenomenon of an emulsion droplet bursting may occur during the manufacturing of the microsphere, and conversely, when the content is lower than 0.1 w/v%, the initial burst of the microsphere including the high content of the tirzepatide may not be sufficiently inhibited.

When the initial burst inhibiting agent is included in the continuous phase during the manufacturing of the microsphere, the initial burst in the microsphere including 12 (w/w)% or more of the tirzepatide may be reduced preferably to 10% or less, more preferably to 8% or less, and most preferably to 5% or less.

The continuous phase used in the step (b) or (b') may further include at least one selected from a group consisting of methyl alcohol, ethyl alcohol, propyl alcohol, and ethyl acetate to control an extraction rate of the organic solvent from the dispersed phase in an emulsion state.

The initial burst inhibiting agent may be added at a time point of preparing the continuous phase in the step (b) or (b'), and the initial burst inhibiting agent may also be added at any time point during supplying of the continuous phase in the step (c) or (c'), and an adding method is not limited.

In addition, the pH of the continuous phase may be 7.0 or more, 7.2 or more, 7.4 or more, 8.0 or more, 8.5 or more, 9.0 or more, 7.0 to 9.0, or 7.0 to 8.5, but it is not limited thereto. When the pH of the continuous phase is controlled to be within the range, the bioavailability of the microsphere including the tirzepatide or the pharmaceutically acceptable salt thereof may be further increased.

In the step (c) or (c'), when the emulsion including the continuous phase that includes the droplet-shaped dispersed phase and the surfactant is maintained or stirred at a temperature below the boiling point of the organic solvent for a certain period of time, for example, 2 hours to 48 hours, the organic solvent may be extracted to the continuous phase from the tirzepatide which is the droplet-shaped dispersed phase or the pharmaceutically acceptable salt thereof and a polymer solution. A part of the organic solvent extracted to the continuous phase may evaporate from a surface thereof. While the organic solvent is extracted and evaporated from the droplet-shaped tirzepatide or the pharmaceutically acceptable salt thereof and the polymer solution, the droplet-shaped dispersed phase may be solidified to form the microsphere.

In order to additionally and efficiently remove the organic solvent in the step (c) or (c'), temperature of the continuous phase may be heated for a certain period of time. The heating temperature is not limited and may be appropriately controlled by those having an ordinary skill in the art depending on the organic solvent that is used. For example, when using dichloromethane (DCM) as the organic solvent, heat may be applied so as to maintain at 30°C or higher, 40 °C or higher, 45 °C or higher, 30 to 50 °C, 40 to 50 °C, or 45 °C.

In the step (d) or (d'), a method of recovering the microsphere including the tirzepatide or the pharmaceutically acceptable salt thereof and the initial burst inhibiting agent may be performed using various known techniques, for example, methods such as filtration or centrifugation may be used.

Between the steps (c) and (d) or between the steps (c') and (d'), the residual surfactant may be removed through filtration and washing, and filtered again to recover the microsphere.

The washing step for removing the residual surfactant may be normally performed using water, and the washing step may be repeated for several times.

In addition, as described above, the sieving process may be additionally used between the steps (c) and (d) or between the steps (c') and (d') to obtain a uniform microsphere. The sieving process may be performed using a known technique, and microspheres with small and large particles may be filtered out using sieves of different sizes to obtain the microsphere with a uniform size.

The method of manufacturing of the present invention may dry the obtained microsphere using a conventional drying method after the step (d) or step (d') or after the filtering and washing step to finally obtain a dried microsphere.

In addition to the above-described matters, all matters including the tirzepatide, the initial burst inhibiting agent, the biodegradable polymer and contents thereof that are not separately defined may be applied as the matters defined in the pharmaceutical composition.

According to the method of manufacturing of the present invention, the sustained-release microsphere injection including the tirzepatide or the pharmaceutically acceptable salt thereof in which the tirzepatide or the pharmaceutically acceptable salt drug thereof is maintained in an effective concentration for a desired period of time without an initial burst of the drug and has a high bioavailability may be manufactured. In addition, the sustained-release microsphere injection with uniform particles having good administration ability including the tirzepatide or the pharmaceutically acceptable salt thereof and the initial burst inhibiting agent may be prepared.

The sustained-release microsphere according to the present invention may be blended with a microsphere including two or more types of drug microspheres including the same drug but having one or more different composition and manufacturing conditions. In a case of blending the microsphere including two or more types of drug microspheres, it may be performed for a purpose of optimizing a release profile of the drug, controlling a release period, and the like.

Specifically, the different composition and manufacturing conditions may be at least one selected from a group consisting of usage amount a drug, polymer type, particle size distribution (e.g., average particle size), circularity, usage amount of a polymer, type and usage amount of a dispersed phase solvent, type and usage amount of co-solvent, type and usage amount of a continuous phase, temperature and time of solidification, theoretical content of a drug, etc., but are not limited thereto.

The microsphere blending may be, for example, mixing the drug microspheres having one or more different composition and manufacturing conditions at a specific ratio.

Specifically, when blending the microsphere, a mixing ratio (weight ratio or ratio of number of microspheres) may be appropriately controlled in consideration of the bioavailability of the active ingredient of each microsphere.

As a specific example, the two or more types of different drug microspheres may be drug microspheres that differ in components and composition ratios (hereinafter, drug microspheres with different compositions) and/or drug microspheres that differ in drug release properties, and for example, at least one selected from a group consisting of the type of a polymer of a microsphere, the content of a polymer, the content of a drug, etc. may be different. The difference in the type of a polymer of the microsphere may be a difference in at least one selected from a group consisting of a repeating unit of a polymer, a terminal group of a polymer, a molecular weight of a polymer, and an intrinsic degree of a polymer, etc.

The microsphere blending may be performed by preparing each microsphere separately and mixing them, or by preparing the microspheres in a single preparing process. Specifically, as an example, an O/W emulsion method of preparing a microsphere blending in a single preparing process may be a preparing method comprising the following steps 1 to 4.
separately preparing two or more types of dispersed phase solutions by dissolving biodegradable polymers and drugs having different compositions in organic solvents, respectively (step 1);
injecting each of the two or more types of dispersed phases prepared in step 1 into an aqueous solution (continuous phase) containing a surfactant to form two or more dispersed phase emulsions (step 2);
extracting and evaporating the organic solvent from the dispersed phase emulsions prepared in the step 2 toward the continuous phase to form microspheres (step 3); and
recovering the microsphere after the step 3 (step 4).

Specifically, as an example, a W/O/W emulsion method of preparing a microsphere blending in a single preparing process may comprising the following steps 1 to 4.

Preparing an aqueous phase (W1 phase) by dissolving a drug in an aqueous solution, preparing two or more types of oil phases (O phase) by dissolving a biodegradable polymer having different compositions in organic solvents, and separately preparing a dispersed phase solution by mixing the aqueous phase (W1 phase) and the two or more types of oil phases (O phase) (step 1);
injecting each of the two or more dispersed phases prepared in Step 1 into an aqueous solution (W2 phase, continuous phase) containing a surfactant to form two or more dispersed phase emulsions (step 2);
extracting and evaporating the organic solvent from the dispersed phase emulsions prepared in step 2 toward the continuous phase (W2 phase) to form a microsphere (step 3); and
recovering the microsphere after the step 3 (step 4).

### [Embodiment of Invention]

### [Examples]

Hereinafter, the present invention will be described in more detail by the following manufacturing examples. However, the following manufacturing examples are merely illustrative of the present invention, and the content of the present invention is not limited by the following manufacturing examples.

### Examples 1-1 to 1-3 (O/W method): Preparing a biodegradable polymer microsphere including tirzepatide and an initial burst inhibiting agent

Tirzepatide (manufacturer: Zhejiang Peptides Biotech Co., Ltd, China) was used as a drug, and RG653H and RG753H (manufacturer: Evonik, Germany) were used as a biodegradable polymer at a weight ratio of 1:1 and weighed so that a batch size becomes 0.5 to 1.5 g (batch size 0.5 g for Examples 1-1 and 1-2, and batch size 1.5 g for Example 1-3). An oil phase (O phase) in which the drug and the polymer are homogeneously dissolved in a mixed solvent of a dichloromethane (DCM) solvent and a glacial acetic acid co-solvent was prepared as a dispersed phase.

Na₂HPO₄ (Disodium phosphate) as an initial burst inhibiting agent was added to a 0.1 (w/v)% polyvinyl alcohol (viscosity: 4.8 to 5.8 mPa.s) aqueous solution to be 2.0 (w/v)% of final concentration and used as a continuous phase (W phase).

After connecting the continuous phase to an emulsifying apparatus equipped with a porous membrane with a diameter of 40 µm, the prepared dispersed phase was injected into the porous membrane together with the continuous phase. An emulsion in which a biodegradable polymer microdroplet including tirzepatide is dispersed was manufactured, and a suspension was put in a preparation vessel and stirred at a speed of 200 to 300 rpm.

A temperature of the preparation vessel was maintained at 25 °C, and after injection of the dispersed phase was completed, the organic solvent was removed while maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

**[Table 1]**

| Example No. | T/L % | Polymer type | Usage amount of polymer (g) | Usage amount of tirzepatide (g) | Usage amount of DCM (g) | Usage amount of glacial acetic acid (g) | Initial burst inhibiting agent and concent ration |
|---|---|---|---|---|---|---|---|
| 1-1 | 15 | RG653H:RG753H=1:1 | 0.425 | 0.075 | 5.24 | 2.81 | 2(w/v)% Na₂HPO ₄ |
| 1-2 | 20 | RG653H:RG753H=1:1 | 0.400 | 0.100 | 4.93 | 2.76 | 2(w/v)% Na₂HPO ₄ |
| 1-3 | 15 | RG653H:RG753H=1:1 | 1.275 | 0.225 | 15.70 | 8.51 | 2(w/v)% Na₂HPO ₄ |

| | | | | | | | |
|---|---|---|---|---|---|---|---|
| * T/L: Target Loading | | | | | | | |

### Examples 2-1 to 2-9 (O/W method): Preparing a biodegradable polymer microsphere including tirzepatide and an initial burst inhibiting agent (difference in type of the initial burst inhibiting agent or concentration)

Tirzepatide (manufacturer: Chengdu Shengnuo Biopharm Co., Ltd, China) was used as a drug, and RG653H and RG753H (manufacturer: Evonik, Germany) were used as a biodegradable polymer at a weight ratio of 1:1 and weighed so that a batch size becomes 0.5g (Target Loading 20%). An oil phase (O phase) in which the drug and the polymer are homogeneously dissolved in a mixed solvent of a dichloromethane (DCM) solvent and a glacial acetic acid co-solvent was prepared as a dispersed phase.

An initial burst inhibiting agent was added to a 0.1 (w/v)% polyvinyl alcohol (viscosity: 4.8 to 5.8 mPa.s) aqueous solution to be a final concentration and used as a continuous phase (W phase).

After connecting the continuous phase to an emulsifying apparatus equipped with a porous membrane with a diameter of 40 µm, the prepared dispersed phase was injected into the porous membrane together with the continuous phase. An emulsion in which a biodegradable polymer microdroplet including tirzepatide is dispersed was manufactured, and a suspension was put in a preparation vessel and stirred at a speed of 200 to 300 rpm.

A temperature of the preparation vessel was maintained at 25 °C, and after injection of the dispersed phase was completed, the organic solvent was removed while maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

**[Table 2]**

| Example No. | Polymer type | Usage amount of polymer (g) | Usage amount of tirzepatide (g) | Usage amount of DCM (g) | Usage amount of glacial acetic acid (g) | Initial burst inhibiting agent and concentration (w/v)% |
|---|---|---|---|---|---|---|
| 2-1 | RG653H:RG753H=1: 1 | 0.400 | 0.100 | 4.93 | 2.94 | 1% K₂HPO₄ |
| 2-2 | RG653H:RG753H=1: 1 | 0.400 | 0.100 | 4.93 | 3.00 | 1% Na₂CO₃ |
| 2-3 | RG653H:RG753H=1: 1 | 0.400 | 0.100 | 4.93 | 3.00 | 1% (NH₄)₂HPO₄ |
| 2-4 | RG653H:RG753H=1: 1 | 0.400 | 0.100 | 4.93 | 3.00 | 1% NaHCO₃ |
| 2-5 | RG653H:RG753H=1: 1 | 0.400 | 0.100 | 4.93 | 2.94 | 1% Na₂HPO₄ |
| 2-6 | RG653H:RG753H=1: 1 | 0.400 | 0.100 | 4.93 | 3.00 | 2% K₂HPO₄ |
| 2-7 | RG653H:RG753H=1: 1 | 0.400 | 0.100 | 4.93 | 3.02 | 2% Na₂CO₃ |
| 2-8 | RG653H:RG753H=1: 1 | 0.400 | 0.100 | 4.93 | 3.03 | 2% (NH₄)₂HPO₄ |
| 2-9 | RG653H:RG753H=1: 1 | 0.400 | 0.100 | 4.93 | 3.03 | 2% NaHCO₃ |

### Examples 3-1 to 3-2 (O/W method): Preparing a biodegradable polymer microsphere including tirzepatide and initial burst inhibiting agent (difference in type of a polymer)

Tirzepatide (manufacturer: Chengdu Shengnuo Biopharm Co., Ltd, China) was used as a drug, and RG503H and RG203H (manufacturer: Evonik, Germany) were used as a biodegradable polymer and weighed so that a batch size becomes 0.5g (Target Loading 20%). An oil phase (O phase) in which the drug and the polymer are homogeneously dissolved in a mixed solvent of a dichloromethane (DCM) solvent and a glacial acetic acid co-solvent was prepared as a dispersed phase.

Na₂HPO₄ (Disodium phosphate) as an initial burst inhibiting agent was added to a 0.1 (w/v)% polyvinyl alcohol (viscosity: 4.8 to 5.8 mPa.s) aqueous solution to be 2.0 (w/v)% of final concentration and used as a continuous phase (W phase).

After connecting the continuous phase to an emulsifying apparatus equipped with a porous membrane with a diameter of 40 µm, the prepared dispersed phase was injected into the porous membrane together with the continuous phase. An emulsion in which a biodegradable polymer microdroplet including tirzepatide is dispersed was manufactured, and a suspension was put in a preparation vessel and stirred at a speed of 200 to 300 rpm.

A temperature of the preparation vessel was maintained at 25 °C, and after injection of the dispersed phase was completed, the organic solvent was removed while maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

**[Table 3]**

| Example No. | Polymer type | Usage amount of polymer (g) | Usage amount of tirzepatide (g) | Usage amount of DCM (g) | Usage amount of glacial acetic acid (g) | Initial burst inhibiting agent and concentration |
|---|---|---|---|---|---|---|
| 3-1 | RG503H | 0.400 | 0.100 | 4.93 | 3.00 | 2(w/v)% Na₂HPO₄ |
| 3-2 | R203H | 0.400 | 0.100 | 4.93 | 3.06 | 2(w/v)% Na₂HPO₄ |

### Examples 4-2 to 4-3 (W/O/W method): Preparing a biodegradable polymer microsphere including tirzepatide and an initial burst inhibiting agent (difference in a content of drug loading or type of a polymer)

As a drug, tirzepatide (Chengdu Shengnuo Biopharm Co., Ltd, China) was dissolved in tertiary distilled water to manufacture a primary aqueous phase. An oil phase (O phase) was manufactured by using RG653H and RG753H (manufacturer: Evonik, Germany) as a biodegradable polymer at a weight ratio of 1:1, or by using RG503H (manufacturer: Evonik, Germany) alone and dissolving the same in dichloromethane (DCM). An aqueous phase was dissolved in the oil phase using a homogenizer to manufacture a primary W1/O emulsion (dispersed phase).

Na₂HPO₄ (Disodium phosphate) as an initial burst inhibiting agent was added to a 0.1 (w/v)% polyvinyl alcohol (viscosity: 4.8 to 5.8 mPa.s) aqueous solution to be 2.0 (w/v)% of final concentration and used as a continuous phase (W2 phase).

After connecting the continuous phase to an emulsifying apparatus equipped with a porous membrane with a diameter of 40 µm, the prepared dispersed phase was injected into the porous membrane together with the continuous phase. A secondary W1/O/W2 emulsion in which a biodegradable polymer microdroplet including tirzepatide is dispersed was manufactured, and a suspension was put in a preparation vessel and stirred at a speed of 200 to 300 rpm.

A temperature of the preparation vessel was maintained at 25 °C, and after injection of the dispersed phase was completed, a temperature of the suspension was maintained at 40 °C for 3 hours and the organic solvent was removed. Thereafter, the suspension was cooled to a temperature of 25 °C, filtered to remove residual polyvinyl alcohol with tertiary distilled water, and then freeze-dried.

**[Table 4]**

| Example No. | T/L % | Polymer type | Usage amount of polymer (g) | Usage amount of tirzepatide (g) | Usage amount of DCM (g) | Weight ratio of DW:DC M | Initial burst inhibiting agent and concentration |
|---|---|---|---|---|---|---|---|
| 4-2 | 20 | RG653H:RG75 3H=1:1 | 0.400 | 0.100 | 7.6 | 1:10.4 | 2(w/v)% Na₂HPO₄ |
| 4-3 | 30 | RG503H | 0.350 | 0.150 | 11.3 | 1:9.3 | 2(w/v)% Na₂HPO₄ |

### Comparative Example 1 (O/W method): Preparing a biodegradable polymer microsphere including tirzepatide without including an initial burst inhibiting agent

0.045g of tirzepatide (manufacturer: Zhejiang Peptides Biotech Co.Ltd, China) was used as a drug, and 0.255g of RG653H and RG753H (manufacturer: Evonik, Germany) were used as a biodegradable polymer at a weight ratio of 1:1 and weighed so that a batch size becomes 0.3g (Target Loading 15%). Dichloromethane (DCM) was used as a solvent for manufacturing a dispersed phase, and glacial acetic acid was used as a co-solvent to homogeneously dissolve the same.

2,000ml of 0.1% polyvinyl alcohol (viscosity: 4.8~5.8mPa.s) aqueous solution was used as a continuous phase.

After connecting the continuous phase to an emulsifying apparatus equipped with a porous membrane with a diameter of 40 µm, the prepared dispersed phase was injected into the porous membrane together with the continuous phase. An emulsion in which a biodegradable polymer microdroplet including tirzepatide is dispersed was manufactured, and a suspension was put in a preparation vessel and stirred at a speed of 200 to 300 rpm.

A temperature of the preparation vessel was maintained at 25 °C, and after injection of the dispersed phase was completed, the organic solvent was removed while maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

**[Table 5]**

| Comparative Example No. | Polymer type | Usage amount of polymer (g) | Usage amount of tirzepatide (g) | Usage amount of DCM (g) | Usage amount of glacial acetic acid (g) |
|---|---|---|---|---|---|
| 1 | RG653H:RG75 3H=1:1 | 0.255 | 0.045 | 3.1 | 1.69 |

### Experimental Example 1. Measuring an encapsulation amount of tirzepatide inside a microsphere

In order to measure an encapsulation amount of tirzepatide of the microsphere manufactured in Examples and Comparative Examples, 10 mg of the microsphere was completely dissolved in DMSO. 20 µL of a diluted solution was injected into HPLC and measured at a detection wavelength of 280 nm. A column used in the present Experimental Example is ZORBAX 300SB-C18, 5 µm, 4.6 x 150 mm, and acetonitrile including 0.1% (w/w) trifluoroacetic acid and a 0.1% (w/w) trifluoroacetic acid aqueous solution was used by mixing in a ratio of 40:60 (v/v).

**[Table 6]**

| | **Encapsulation rate (%)** | **Drug content(wt%)** |
|---|---|---|
| Example 1-1 | 74.80 | 11.22 |
| Example 1-2 | 75.70 | 15.14 |
| Example 1-3 | 77.20 | 11.58 |
| Example 2-1 | 55.12 | 11.02 |
| Example 2-2 | 57.47 | 11.49 |
| Example 2-3 | 61.93 | 12.39 |
| Example 2-4 | 53.90 | 10.78 |
| Example 2-5 | 55.12 | 11.02 |
| Example 2-6 | 55.04 | 11.01 |
| Example 2-7 | 65.74 | 13.15 |
| Example 2-8 | 63.01 | 12.60 |
| Example 2-9 | 59.11 | 11.82 |
| Example 3-1 | 57.06 | 11.41 |
| Example 3-2 | 51.25 | 10.25 |
| Example 4-2 | 70.65 | 14.13 |
| Example 4-3 | 76.55 | 22.97 |
| Comparative Example 1 | 103.20 | 15.48 |

As shown in Table 6,
when viewing a result of Examples 1-1 to 1-3 manufactured with the O/W method, it may be confirmed that the encapsulation rate is maintained at 70% or more up to T/L 20%,
when viewing a result of Examples 2-1 to 2-9 manufactured with the O/W method but differed in a type of the initial burst inhibiting agent or concentration, it may be confirmed that the encapsulation rate is maintained between 50 and 70%,
when viewing a result of Examples 3-1 and 3-2 manufactured with the O/W method but differed in a type of the polymer, it may be confirmed that the encapsulation rate is maintained between 50 and 70%, and
viewing a result of Examples 4-2 and 4-3 a loading content of the drug or a type of the polymer type differs in the W/O/W method, it may be confirmed that the encapsulation rate is maintained at 70% or more up to T/L 30%.

### Experimental Example 2. Measuring initial burst of a drug of a tirzepatide microsphere in vitro

In order to confirm an initial burst of a drug from the microsphere manufactured in Examples and Comparative Examples, the following experiment was performed. 10 mg of the microsphere was put in an HDPE wide-mouth bottle, filled with 50 mL of a release test solution, and stored in a 37 °C incubator. After 24 hours, a supernatant obtained by taking and centrifuging 1 mL of the test solution was analyzed for tirzepatide content and release rate using HPLC under the same analysis conditions as in Experimental Example 1. The release test solution used for this measurement was a pH 7.4 PBS solution including sodium dodecyl sulfate and azide sodium.

**[Table 7]**

| | **In vitro** |
|---|---|
| | **Initial burst rate(%, 1-day)** |
| Example 1-1 | 1.60 |
| Example 1-2 | 2.94 |
| Example 1-3 | 1.85 |
| Example 2-1 | 1.74 |
| Example 2-2 | 3.55 |
| Example 2-4 | 1.66 |
| Example 2-5 | 2.03 |
| Example 2-6 | 1.28 |
| Example 2-7 | 1.85 |
| Example 2-8 | 2.48 |
| Example 2-9 | 1.12 |
| Example 3-1 | 4.13 |
| Example 3-2 | 3.57 |
| Example 4-2 | 7.75 |
| Comparative Example 1 | 44.59 |

As shown in Table 7, it was confirmed that the initial burst of the drug from the microsphere including the tirzepatide was significantly reduced as the initial burst inhibiting agent was included in the continuous phase during the manufacturing of the microsphere. Meanwhile, in a case of Comparative Example 1 that did not include the initial burst inhibiting agent, a day 1 release rate was 44.59%, confirming that the initial burst occurred.

### Experimental Example 3. Morphological analysis through electron microscope

Morphological features of the microsphere according to the present invention were analyzed using an electron microscope. The experimental procedure is as follows. 5 mg of the microsphere manufactured in Examples and Manufacturing Examples was put on an aluminum stub on which a carbon tape is attached and coated with platinum using an ION-COATER (COXEM, Korea). The aluminum stub was equipped on a scanning electron microscope (COXEM EM-30, Korea) and the morphological features of the microsphere were observed at an acceleration voltage of 10 to kV and a result is shown in FIGS. 1A (Example 1-1) and 1B (Comparative Example 1).

FIG. 1A is a scanning electron microscope photograph confirming the morphological features of the microsphere manufactured in Example 1-1.

FIG. 1B is a scanning electron microscope photograph confirming the morphological features of the microsphere manufactured in Comparative Example 1.

### Experimental Example 4. Evaluation of in-vivo pharmacokinetics using rats

In order to evaluate an in-vivo drug release pattern of the microsphere according to an embodiment of the present invention, a concentration of tirzepatide in blood was measured after administration to rats.

The microsphere was measured to be 3.6 mg/head (12.0 mg/kg) as the tirzepatide, dispersed in 0.5 mL of a suspension, and then subcutaneously injected into Sprague-Dawley (SD) rats (300 g). 0.5 mL of blood was collected at pre-scheduled times and the concentration of the tirzepatide in blood was measured using HPLC.

**[Table 8]**

| Experiment no. | **G2** | **G4** | **G6** | **G7** |
|---|---|---|---|---|
| Example no. | 1-1 | 1-2 | Comparative Example 1 | 1-3 |
| T/L % | 15 | 20 | 15 | 15 |
| Dose(mg/head) | 3.6 | 3.6 | 3.6 | 3.6 |
| AUCnorm (ng*day/m L)/(mg/head) | 5422.0 | 2670.8 | 3854.5 | 3170.6 |
| Cmax(ng/mL) | 1503.7 | 994.1 | 7217.5 | 939.3 |

As shown in Table 8, it may be confirmed that the bioavailability of the tirzepatide microsphere manufactured in the Examples was at a good level.

### Experimental Example 5. Experiment of pharmacokinetics of a single-dose subcutaneous administration using SD rats

In order to evaluate a potential of the microsphere according to the present invention as a sustained-release therapeutic agent, the concentration of the tirzepatide in rat blood was measured by the following method.

Specifically, the microsphere was measured so that a dosage of the tirzepatide becomes 3.6 mg/head, dispersed in 0.5 mL of a suspension, and then subcutaneously injected into SD rats. 0.25 to 0.5 mL of blood was collected at pre-scheduled times and the concentration of the tirzepatide in blood was measured using HPLC.

**[Table 9]**

| Time(day)-Concetration (ng/mL) | | | | |
|---|---|---|---|---|
| Time(day) | Example no. | | | Comparative Example no. |
| | 1-1 | 1-2 | 1-3 | 1 |
| 0 | 0.0 | 0.0 | 0.0 | 0.0 |
| 0.25 | 223.9 | 548.2 | 685.7 | 3791.4 |
| 1 | 464.3 | 994.1 | 939.3 | 7217.5 |
| 4 | 22.4 | 111.4 | 751.6 | 243.6 |
| 7 | 250.6 | 205.5 | 488.4 | 391.1 |
| 10 | 509.1 | 102.4 | 600.9 | 151.2 |
| 14 | 1503.7 | 86.1 | 202.9 | 62.2 |
| 17 | 916.7 | 113.7 | 101.4 | 53.9 |
| 21 | 189.1 | 110.9 | 71.0 | 34.9 |
| 28 | 321.6 | 287.1 | 43.4 | 20.4 |
| 35 | 486.5 | 391.3 | 169.3 | 28.2 |
| 42 | 232.3 | 116.6 | 99.8 | 14.9 |
| 49 | 44.4 | 16.8 | 32.5 | 10.5 |

As shown in Table 9, the microsphere prepared in Examples showed an excellent sustained-release profile with no initial burst until day 1 and continuously release until day 49. On the other hand, it was confirmed that the microsphere of Comparative Example 1 that did not include the initial burst inhibiting agent exhibited an initial burst until day 1, thereby releasing most of the drug and releasing at an insignificant concentration from day 10 onwards.

### Experimental Example 6. Measuring a residual content of an initial burst inhibiting agent in a microsphere

In order to measure a content of an initial burst inhibiting agent in the microsphere manufactured in Examples, a residual amount of sodium (Na) and phosphorus (P) in the microsphere was measured.

Specifically, 300 mg of the microsphere was mixed with 6 mL of a nitric acid aqueous solution and 3 mL of hydrogen peroxide mixed with ultrapure water in a 1:1 ratio, heated at 100 °C or higher, and an acid was added until a gas generated during the dissolution process changed from yellow to white. A sample obtained through this was weighed, dissolved in ultrapure water, and then injected into an inductively coupled plasma optical emission spectrometer (ICP-OES) (Thermo Scientific Co., iCAP 6300 Duo, UK) and measured at a detection wavelength of 598.5 nm.

**[Table 10]**

| | Example 3-2 | Example 1-1 | Example 4-2 | Example 2-6 |
|---|---|---|---|---|
| Initial burst inhibiting agent | 2(w/v)% | 2(w/v)% | 2.0(w/v)% | 2(w/v)% |
| | Na₂HPO₄ | Na₂HPO₄ | Na₂HPO₄ | K₂HPO₄ |
| Polymer | R203H | RG 653H:RG 753H | RG 653H:RG 753H | RG 653H:RG 753H |
| Residual Sodium (mg/kg) | 388 | 102 | 121 | - |
| Residual Phosphorus (mg/kg) | 80 | 35 | 44 | 42 |

As shown in Table 10, a content of residual sodium and phosphorus according to the initial burst inhibiting agent included in the continuous phase differs depending on a manufacturing condition of the microsphere, and a content of the initial burst inhibiting agent remaining in the microsphere calculated from this was confirmed to be 50 ppm to 500 ppm based on sodium (Na) and 10 to 100 ppm based on phosphorus (P).

## Claims

1. A pharmaceutical composition for preventing or treating diabetes, type 2 diabetes, beta-cell dysfunction, obesity, or non-alcoholic steatohepatitis, the pharmaceutical composition comprising:
sustained-release microspheres consisting of tirzepatide or a pharmaceutically acceptable salt thereof, an initial burst inhibiting agent, and a biodegradable polymer
wherein the tirzepatide or the pharmaceutically acceptable salt thereof is included as the tirzepatide in an amount of 8 wt.% or more based on a total weight of the microsphere, and
an amount of the initial burst inhibiting agent is included in 5 ppm to 2000 ppm.

2. The pharmaceutical composition of claim 1, wherein the sustained-release microsphere including the tirzepatide or the pharmaceutically acceptable salt thereof and the initial burst inhibiting agent releases less than 20% of the tirzepatide or the pharmaceutically acceptable salt thereof for 24 hours when administered in vivo.

3. The pharmaceutical composition of claim 1, wherein the sustained-release microsphere including the tirzepatide or the pharmaceutically acceptable salt thereof and the initial burst inhibiting agent releases less than 15% of the tirzepatide or the pharmaceutically acceptable salt thereof within 24 hours when administered in vivo.

4. The pharmaceutical composition of claim 1, wherein the sustained-release microsphere containing the tirzepatide or the pharmaceutically acceptable salt thereof and the initial burst inhibiting agent releases less than 10% of the tirzepatide or the pharmaceutically acceptable salt thereof within 24 hours when administered in vivo.

5. The pharmaceutical composition of claim 1, wherein the biodegradable polymer is at least one selected from a group consisting of:
a polymer selected from a group consisting of polylactide (PLA), polyglycolide (PGA), polylactide-co-glycolide (PLGA), polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly(butylene succinate lactide) (PBSLA); a copolymer or a simple mixture of two or more types selected from said polymer; a copolymer with said selected polymer and polyethylenglycol (PEG); and a polymer-sugar complex in which the said selected polymer or the copolymer and sugar are bonded.

6. The pharmaceutical composition of claim 1, wherein the initial burst inhibiting agent is one or more types of substances selected from phosphate salt, hydroxide salt, phosphide salt, phosphite salt, carbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt, and nitride salt of an alkali metal, an alkaline-earth metal, and ammonium.

7. The pharmaceutical composition of claim 1, wherein an average particle size of the microsphere including the tirzepatide or the pharmaceutically acceptable salt thereof and the initial burst inhibiting agent is 5 µm to 100 µm.

8. The pharmaceutical composition of claim 1, wherein a total weight of the microsphere including the tirzepatide or the pharmaceutically acceptable salt thereof and the initial burst inhibiting agent is from 20 mg to 3000 mg.

9. The pharmaceutical composition of claim 1, wherein an intrinsic viscosity of the biodegradable polymer is 0.16 dL/g to 1.7 dL/g.

10. The pharmaceutical composition of claim 1, wherein the microsphere further includes one or more types of release control agents selected from a group consisting of butyric acid, valeric acid, caproic acid, enantic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, behenic acid, arachidic acid, isocrotonic acid, oleic acid, elaidic acid, sorbic acid, linoleic acid, arachidonic acid, benzoic acid, hydroxynaphthoic acid, napadisylic acid, naphthalenesulfonic acid, and pamoic acid.

11. A method of preparing a microsphere with an oil-in-water (O/W) emulsion comprising tirzepatide or a pharmaceutically acceptable salt thereof, an initial burst inhibiting agent, and a biodegradable polymer, the method comprising:
(a) preparing an oil phase (O phase) as a dispersed phase obtained by dissolving tirzepatide or a pharmaceutically acceptable salt thereof and one or more types of biodegradable polymers in an organic solvent;
(b) preparing a continuous phase (W phase) by adding an initial burst inhibiting agent to an aqueous solution including a surfactant, and then adding the dispersed phase of the step (a) to form an emulsion state of the dispersed phase;
(c) extracting the organic solvent from the dispersed phase in the emulsion state obtained in the step (b) to the continuous phase (W phase) and evaporating the organic solvent to form microspheres; and
(d) recovering the microsphere.

12. A method of preparing a microsphere with a water-in-oil-in-water (W/O/W) emulsion comprising tirzepatide or a pharmaceutically acceptable salt thereof, an initial burst inhibiting agent, and a biodegradable polymer, the method comprising:
(a') preparing a primary aqueous phase (W1 phase) by dissolving tirzepatide or a pharmaceutically acceptable salt thereof in an aqueous solution and preparing an oil phase (O phase) by dissolving one or more of biodegradable polymers in an organic solvent to prepare a primary W1/O emulsion as a dispersed phase in which the aqueous phase (W1 phase) and the oil phase (O phase) are mixed;
(b') preparing a continuous phase (W2 phase) by adding an initial burst inhibiting agent to an aqueous solution including a surfactant, and then adding the dispersed phase of the step (a') to prepare a dispersed phase in a secondary W1/O/W2 emulsion state;
(c') extracting an organic solvent from the dispersed phase in the secondary W1/O/W2 emulsion state of the step (b') to the continuous phase (W2 phase) and/or evaporating the organic solvent to form a microsphere; and
(d') recovering the microsphere.

13. The method of any one of claim 11 and claim 12, wherein the biodegradable polymer is at least one selected from a group consisting of: a polymer selected from a group consisting of polylactide (PLA), polyglycolide (PGA), polylactide-co-glycolide (PLGA), polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly(butylene succinate lactide) (PBSLA); a copolymer or a simple mixture of two or more types selected from said polymer; a copolymer with said selected polymer and polyethylenglycol (PEG); and a polymer-sugar complex in which said selected polymer or the copolymer and sugar are bonded.

14. The method of any one of claim 11 and claim 12, wherein the dispersed phase of the step (a) or (a') further includes one or more types of release control agents selected from a group consisting of butyric acid, valeric acid, caproic acid, enantic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, arachidic acid, isocrotonic acid, oleic acid, elaidic acid, sorbic acid, linoleic acid, arachidonic acid, hydroxynaphthoic acid, napadisylic acid, and pamoic acid.

15. The method of any one of claim 11 and claim 12, wherein the organic solvent of the step (a) or (a') is one or more types of organic solvents selected from a group consisting of dichloromethane (DCM), chloroform, ethyl acetate, methyl ethyl ketone, acetone, acetonitrile, dimethyl sulfoxide, dimethyl formamide, n-methyl pyrrolidone, acetic acid, methyl alcohol, ethyl alcohol, propyl alcohol, and benzyl alcohol.

16. The method of any one of claim 11 and claim 12, wherein the surfactant of the step (b) or (b') is polyvinyl alcohol.

17. The method of any one of claim 11 and claim 12, wherein a continuous phase of the step (b) or (b') further includes at least one selected from a group consisting of methyl alcohol, ethyl alcohol, propyl alcohol, and ethyl acetate.

18. The method of any one of claim 11 and claim 12, wherein the continuous phase of the step (b) is used by adding the initial burst inhibiting agent to the aqueous solution including the surfactant to a final concentration of 0.1 to 5.0 (w/v)%.

19. The method of any one of claim 11 and claim 12, wherein the initial burst inhibiting agent is at least one selected from a group consisting of phosphate salt of two or more alkali metals, carbonate salt of one or more alkali metals, and phosphate salt of two or more ammoniums.

20. The method of any one of claim 11 and claim 12, wherein a pH of the continuous phase of the step (b) or (b') is 7 or more.

21. The method of any one of claim 11 and claim 12, wherein a release rate of the tirzepatide or the pharmaceutically acceptable salt thereof is less than 15% within 24 hours of when a prepared sustained-release microsphere including the tirzepatide or the pharmaceutically acceptable salt thereof and the initial burst inhibiting agent is administered in vivo.

22. The method of any one of claim 11 and claim 12, wherein a release rate of the tirzepatide or the pharmaceutically acceptable salt thereof is less than 10% within 24 hours when a prepared sustained-release microsphere including the tirzepatide or the pharmaceutically acceptable salt thereof and the initial burst inhibiting agent is administered in vivo.
